Europäisches Patentamt

European Patent Office

Office européen des brevets

(11) Publication number: **0 267 955 B1**

(12) EUROPEAN PATENT SPECIFICATION

(45) Date of publication of patent specification: **08.07.92**  (51) Int. Cl.⁵: **A61M 5/14**

(21) Application number: **87903942.8**

(22) Date of filing: **28.05.87**

(86) International application number:
**PCT/US87/01206**

(87) International publication number:
**WO 87/07158 (03.12.87 87/27)**

Divisional application 91201018.8 filed on
28/05/87.

(54) DRUG DELIVERY APPARATUS HAVING CHAMBER WITH CHIMNEY.

(30) Priority: **29.05.86 US 868826**

(43) Date of publication of application:
**25.05.88 Bulletin 88/21**

(45) Publication of the grant of the patent:
**08.07.92 Bulletin 92/28**

(84) Designated Contracting States:
**BE CH DE FR GB IT LI NL SE**

(56) References cited:
**EP-A- 0 077 604       EP-A- 0 146 310**
**DE-B- 0 966 701       US-A- 2 612 160**
**US-A- 3 208 639       US-A- 3 993 066**
**US-A- 4 515 585       US-A- 4 552 555**
**US-A- 4 581 016       US-A- 4 589 867**
**US-A- 4 614 437**

(73) Proprietor: **BAXTER INTERNATIONAL INC. (a
Delaware corporation)
One Baxter Parkway
Deerfield Illinois 60015(US)**

(72) Inventor: **ZDEB, Brian, D.
2 East Lakeshore
Round Lake Park, IL 60073(US)**
Inventor: **YOUNKES, William, E.
1751 White Fence Lane
Libertyville, IL 60048(US)**
Inventor: **ROESER, Thomas, J.
1209 Lake Shore Drive
Barrington, IL 60010(US)**

(74) Representative: **MacGregor, Gordon et al
ERIC POTTER & CLARKSON St. Mary's Court
St. Mary's Gate
Nottingham, NG1 1LE(GB)**

EP 0 267 955 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

### Technical Field of the Invention

The present invention is related to apparatus for the delivery of a beneficial agent to a patient and is more particularly directed to apparatus for the passive delivery of the beneficial agent to the intravenous system of a patient in a safe and effective manner.

### Background of the Invention

Many drugs are mixed with a diluent before being delivered intravenously to a patient. The diluent may be, for example, a dextrose solution, a saline solution or even water. Many such drugs are supplied in powder form and packaged in glass vials or ampules. Other drugs, such as some used in chemotherapy, are packaged in glass vials or ampules in a liquid state.

Powdered drugs may be reconstituted in a well known manner, utilizing a syringe which is used to inject liquid into the vial for mixing, the syringe eventually withdrawing the mixed solution from the vial. When a drug must be diluted before delivery to a patient the drug is often injected into a container of diluent after it is reconstituted, where the container may be connected to an administration set for delivery to a patient. More specifically, the diluent is often packaged in glass bottles, or flexible plastic containers such as are sold under the names MINI-BAG™ AND VIAFLEX® by Travenol Laboratories, Inc. of Deerfield, Illinois. These containers have administration ports for connection to an administration set which delivers the container contents from the container to the patient. The drug is typically added to the container through an injection site on the container.

Drugs may be packaged separately from the diluent for various reasons. One of the most important reasons is that many drugs do not retain their chemical and physical stability when mixed with a diluent and thus cannot be stored for any substantial period of time. Also, drugs are often packaged separately from the diluent because many firms which manufacture drugs are not engaged in the business of providing medical fluids in containers for intravenous delivery, and vice versa.

Therefore, a doctor, nurse, pharmacist or other medical personnel must mix the drug and diluent. This presents a number of problems. The reconstitution procedure is time consuming and requires aseptic technique. The operator must provide the proper diluent and a syringe before beginning. Often the powdered drug is "caked" at the bottom of the vial. Thus, when liquid is injected into the vial from a syringe the surface area of contact between the liquid and the powdered drug may be quite small initially, thus making the mixing procedure even more time consuming. Because of the limited vial volume, the increasing drug concentration in the diluent makes it harder to finish the reconstitution process. The operator may attempt to solve this by repeatedly injecting solution into the vial, mixing and withdrawing the solution but this makes necessary additional injections and movement of the syringe which increase the likelihood of contamination. Also, it is sometimes difficult to get all of the drug and/or liquid out of the vial, thus increasing the time required to perform the reconstitution procedure.

The reconstitution procedure should be performed under preferably sterile conditions. In addition to such a requirement making the operator justifiably more cautious and consuming more time, sterile conditions are often hard to maintain. In some instances, a laminar flow hood may be required under which the reconstitution procedure is performed.

Some drugs, such as some chemotherapy drugs, are toxic. Exposure of the operator to the drugs during reconstitution may be dangerous, especially if the operator works with such drugs on a daily basis and is repeatedly exposed to them.

A further problem is that the reconstitution procedure provides a source of confusion as to which container contains which drug. The diluent container should be marked with the drug with which it has been injected and the name of the patient to whom it should be delivered.

After a drug is reconstituted and withdrawn into a syringe barrel, the drug may in some instances be injected immediately into the intravenous system of a patient. More typically however, the reconstituted drug is injected from the syringe into a larger container of solution as discussed above, for connection to an intravenous administration set. This is because often the drug reconstituted in the syringe is still at a concentration so high as to cause local toxicity in the veins of a patient near the injection site where the needle pierces the skin. This may create severe vein irritation which may be medically harmful. Additionally, even though the proper dose of medication is in the syringe, immediate injection into the patient's blood stream may create a condition of systemic toxicity wherein the level of drug concentration in the patient's entire blood stream is dangerously high. Yet another reason for not making the injection from the syringe directly into the patient is that it creates an additional injection site into the patient, which may be painful for the patient and provides another opportunity for infection.

For these reasons, the reconstituted drug is more typically injected into a diluent container.

A patient may typically be administered a dextrose or saline solution from a large volume parenteral container, for example, such as a one liter container, delivered through an administration set such as a CONTINU-FLO® administration set sold by Travenol Laboratories. If the reconstituted drug were injected into the large volume parenteral container, delivery of the drug would usually be delivered over too long a time period. Often, these large volume fluids are delivered at very slow flow rates.

More typically, the reconstituted drug is injected into a small volume parenteral container, such as a fifty milliliter container sold by Travenol Laboratories. This MINIBAG™ container is hung at a higher elevation than the large volume parenteral container and is connected by a secondary administration set to an injection site on the primary administration set. Because it is maintained at a higher elevation, the reconstituted drug in the small volume container is delivered, after which fluid from the large volume container begins to flow once more. By utilizing a small volume container connected to an administration set for delivery of the drug or other beneficial agent instead of a direct syringe injection, the drug is delivered over a preferred time period that tends to minimize negative side effects.

A closed reconstitution delivery system is disclosed in U.S. Patent Nos. 4,410,321; 4,411,662; 4,432,755; and 4,458,733, all assigned to Baxter Travenol Laboratories Inc., the assignee of the present invention. As shown therein, a container includes a drug and a diluent in separate compartments which are reconstituted in a closed system before the drug is delivered to the patient. Typically, the container is connected to an administration set which is connected at its other end to the primary administration set, such as with the small volume parenteral container described above. The container shown in these patents solves many of the problems associated with syringe reconstitution. The product does however necessitate a series of reconstitution steps which must be performed by the nurse or other operator prior to delivering the fluid from the container.

Delivery of a drug or other beneficial agent in a manner not requiring reconstitution steps by an operator is shown in U.S. Patent Nos. 4,424,056; 4,432,756; 4,439,183; 4,474,574; 4,479,793; 4,479,794; 4,525,162, and 4,548,599 and Canadian Patent No. 1,173,795, assigned to Alza Corporation of Palo Alto, California. As disclosed in those patents, a parenteral delivery system is disclosed which has a formulation chamber therein for administering a beneficial agent such as a drug. The system is advantageous in that it provides for reconstitution of the drug by fluid flowing from a large volume parenteral container for example, through the administration set containing the formulation chamber with the drug therein. The system intends to eliminate the need for the time consuming reconstitution procedure described above and appears to eliminate the problems associated with the reconstitution procedure.

Another passive reconstitution system is disclosed in European Patent Application No. 0059694 to Aktiebolaget Hassle of Sweden.

Still another device for delivering a drug "in-line", i.e., in the administration set, is disclosed in U.S. Patent No.4,534,757 assigned to Alza Corporation. The device holds the drug and includes a section through which the liquid passes in a direction substantially opposite to the general direction in which liquid flows to the patient.

Yet another system which attempts to provide for drug reconstitution in-line without manual reconstitution by a nurse or other operator is shown in U.S. Patent No. 4,465,471, assigned to Eli Lilly and Co. of Indianapolis, Indiana. That patent discloses constructions for a receptacle in the administration set itself. A separate cartridge containing the drug to be reconstituted and delivered to the patient is plugged into the receptacle. As liquid enters the cartridge for reconstitution of the drug and subsequent delivery out of the cartridge and receptacle and into the patient, some or most fluid continues to flow through the administration set, bypassing the cartridge entirely.

European Patent Application Publication No. 0146310 to Eli Lilly and Co. is directed to a system for drug reconstitution including an intravenous administration set and a drug vial and utilizes the vial vacuum to reconstitute the drug.

U.S. Patent No. 4,534,758 to Akers et al. discloses a relatively complex drug delivery apparatus with various valves. When liquid from a container is delivered to the drug vial, the vial is to be agitated for a time sufficient to suspend the previously dry medicine.

U.S. Patent No. 4,581,014 to Millerd et al. assigned to Ivac Corporation of San Diego, California discloses a selector valve for delivering a previously reconstituted drug from a drug vial through an intravenous administration set to a patient.

All the publications described above are directed to solutions to the time consuming reconstitution procedure and/or its associated problems, such as delivery of the solution to a patient. In most of the offered solutions, delivery of the drug is intended to be passive, i.e., once the drug is placed into the administration set, manual reconstitution steps are not required.

Still another common feature of many of the attempted solutions disclosed in these publications is that delivery of the drug is intended to be able to be made in a manner which is essentially independent of the fluid flow rate through the administration set and into the patient. Stated differently, some of the systems are designed to deliver a certain dosage of drug in a preselected time period, within a broad range of fluid flow rates. Delivery of a drug independent of flow rate is desirable because it ensures that the necessary dosage will be delivered within a therapeutically acceptable time period, which may be typically about twenty to thirty minutes, although this time period may vary depending upon the drug and dosage.

By making delivery of the drug or other beneficial agent independent of the flow rate, the system ensures that the drug will not be delivered too quickly should the flow rate be set too high by the nurse or other operator, thereby preventing the problem of systemic toxicity discussed above.

Some of the documents, such as U.S. Patent Nos. 4,424,056; 4,479,793; and 4,479,794, are also directed to systems having a beneficial agent placed "in-line" in an administration set for mixing of the agent and delivery to a patient, wherein the delivery of the agent may be made in a given volume of fluid. Also, a valve controlling fluid flow may be manually operated to deliver the agent in a manner which can be made dependent upon fluid flow.

It would be desirable to provide a cartridge containing a beneficial agent wherein the cartridge design assures a proper fluid flow path for delivery of the proper amount and concentration of drug to the patient.

The pre-characterising part of Claim 1 is based on the disclosure of EP-A-0146310, referred to above.

The distinguishing features of the invention are set out in the characterising part of Claim 1.

The cartridge may include a flow connector adapted for mounting to the defined chamber and including in one embodiment cartridge connection means, a base mounted to the connection means and two hollow cannulas mounted within the base in a direction substantially parallel to the chimney within the cartridge chamber. The base and the hollow cannulas are slidable relative to the pierceable stopper. In a first position the hollow cannulas are spaced from the chamber with the beneficial agent therein. In a second position the first and second cannulas have both pierced the stopper to the chamber with the second cannula piercing the stopper in alignment with the chimney such that the second cannula is disposed within the chimney. The first cannula pierces the stopper at a point on the inner face of the stopper external to the chimney, thereby creating the desired fluid flow path through the chamber for desired mixing and delivery of the agent to a patient.

Liquid flowing through the administration set enters the receptacle upon which the cartridge is mounted. All liquid entering the receptacle enters the first cannula and flows into the chamber. The liquid level in the chamber rises until it reaches the top of the chimney, whereupon liquid flows down the chimney, out the receptacle and through the remainder of the administration set to the patient.

In the preferred embodiment of the invention the base with the cannulas is rotatable relative to the pierceable closure means and the drug chamber. The pierceable closure means and the base include a mating key and keyway which, when aligned, cause the second cannula to be in alignment with the chimney for piercing of the closure means.

Description of the Drawings

Fig. 1 is a perspective view of an administration set including the air flask and the receptacle;

Fig. 2 is an enlarged, fragmentary, cross-sectional view of the receptacle illustrated in Figure 1;

Fig. 3 is a fragmentary, enlarged, cross-sectional view of the air flask illustrated in Fig. 1;

Fig 4 is a longitudinal cross-sectional view of a cartridge as being inserted into a receptacle having a piston-like injection site;

Fig 5 is a fragmentary cross-sectional view of the cartridge and receptacle of Fig 4 with the injection site having been moved to its stressed position by full engagement of the cartridge and receptacle;

Fig 6 is a cutaway, perspective view of a cartridge which, in combination with an administration set, is in accordance with the present invention;

Fig 7 is a longitudinal cross-sectional view of the cartridge of Fig 6;

Fig 8 is a cross-sectional view of the cartridge of Fig 8 after flow communication with a chamber has been effected; and

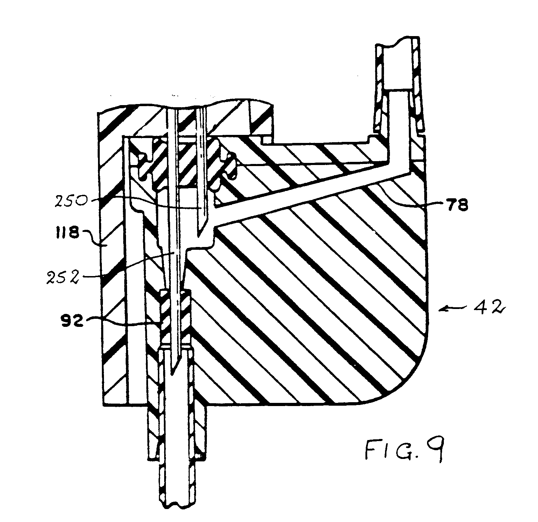

Fig 9 is a cross-sectional view of the receptacle of Fig 2 showing connection with cannulas of a cartridge.

Detailed Description

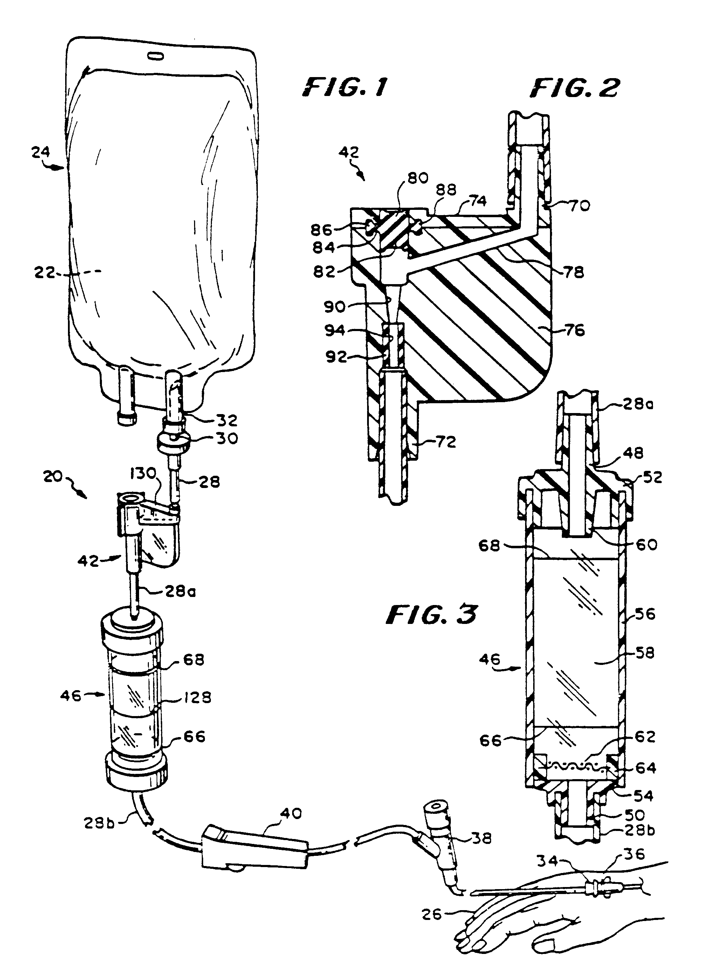

Referring to Fig. 1, there is illustrated an administration set 20 for the delivery of a medical liquid, stored within a medical liquid source such as large volume parenteral container 24, to a patient 26. The administration set 20 includes a fluid conduit 28 made for example of flexible polyvinylchloride tubing. Upstream connection means such as a standard intravenous administration set spike 30 is mounted at the upstream end of the fluid conduit 28. The spike is adapted for piercing the membrane of the container administration port 32.

The fluid conduit 28 includes downstream connection means such as a Luer taper 34 mounted at the downstream end of the fluid conduit 28. The Luer taper 34 may be connected in accordance with standard technique to a venous catheter 36.

The administration set 20 may further include a standard pierceable injection site 38 for injecting a medical liquid by means of a needle through the injection site 38. The administration set 20 may further include flow rate control means such as a standard roller clamp 40 mounted about the flow conduit 28.

The administration set 20 further includes a receptacle 42 shown in greater detail in Fig. 2. The receptacle 42 is mounted along the fluid conduit and is adapted for receiving a separate cartridge 208 containing beneficial agent. When the cartridge is mounted upon the receptacle, at least some, and preferably all liquid from the medical liquid source container 24 that flows through the fluid conduit 28 into the receptacle 42 also flows through the cartridge 208 before passing downstream out of the receptacle to the patient.

Downstream of the receptacle 42 is an air chamber 46, illustrated in Figs. 1 and 3. As will be explained in greater detail below, the air flask 46 permits automatic priming of the cartridge 208 upon mounting of the cartridge on the receptacle 42 of the administration set 20. The air flask 46 absorbs the air disposed within the cartridge 208 and prevents that air from passing downstream to the patient.

Referring to Fig. 3, the air flask 46 includes an inlet 48 mounted to and receiving fluid from upstream fluid conduit 28a. The air flask 46 includes an outlet 50 mounted to and transferring liquid to downstream fluid conduit 28b. The inlet and outlet may be mounted to the fluid conduit 28 by means of interference fit, solvent bonding etc. The air flask 46 is mounted downstream of the receptacle 42.

In the preferred embodiment the air flask 42 includes inlet and outlet end caps 52, 54 respectively between which is mounted a cylindrical sidewall 56 of preferably optically transparent, flexible material such as polyvinyl chloride . The sidewall 56 and the end caps 52, 54 define an air chamber 58 having a cross-sectional diameter that is greater than the internal diameter of the fluid conduit 28, so that liquid entering the air chamber 58 from the drop forming orifice 60 adjacent the inlet 48 falls toward the outlet 50. The air flask 46 thus provides a collection reservoir for air within the administration set 20.

The air flask 46 further includes particulate matter barrier means such as a particulate matter screen 62 mounted within a plastic ring 64 near the outlet 50. The particulate matter barrier may in fact be a sterilizing filter having a nominal pore size of about 0.2 micron. The nominal pore size may be much larger, such as a gross particulate matter barrier having a nominal pore size of about 20 microns. In the preferred embodiment the nominal pore size is about 10 microns. The screen may be a nylon mesh material such as supplied by Filter Tek of Hebron, Illinois. The particulate matter barrier 62 is mounted transverse to the fluid path such that all liquid passing through the air flask 46 must pass through the particulate matter barrier 62.

The particulate matter barrier 62 need not be disposed within the air flask 46 but the barrier should be mounted downstream of the receptacle 42 so that all liquid that exits the inserted cartridge 208 will pass through the particulate matter barrier. Also, it is possible to construct the receptacle 42, the air flask 46 and the particulate matter barrier 62 as a single unit rather than as separated by upstream fluid conduit 28a for example.

In the preferred embodiment, the air flask 46 includes a minimum liquid level indicator 66 and a maximum liquid level indicator 68 which may for example comprise lines around the periphery of the air flask 46. The liquid level in the air flask 46 should preferably be somewhere between the minimum and maximum liquid level indicators 66, 68 immediately before insertion of the cartridge 208 within the receptacle 42.

The improved receptacle 42 includes a receptacle inlet 70 and a receptacle outlet 72 connected to the fluid conduit 28. The air flask 46 is disposed downstream of the receptacle outlet 72.

The receptacle 42 includes upper and lower fitments 74, 76 respectively. The upper fitment 74 includes the inlet. The lower fitment 76 includes the outlet 72 and a fluid receiving segment 78 having an upstream end in fluid communication with the inlet 70 and a downstream end in fluid communication with the outlet 72.

Pierceable situs 80 is mounted within the receptacle 42, trapped between the upper and lower fitments 74, 76. The pierceable situs 80 includes a pierceable main body portion 82 and a ring-like extension 84 extending about the periphery of the main body portion 82. The ring-like extension 84 further includes an enlarged outer periphery 86.

Together, the upper and lower fitments 74, 76 define an annular channel 88 substantially corresponding to and receiving the ring-like extension 84 including the enlarged periphery 86 thereof, such that the upper and lower fitments trap the pierceable situs 80 therebetween in secure fashion. The situs 80 may not be removed without destruction of the receptacle 42. The upper and lower fitments 74, 76 may be bonded together by adhesive, ultrasonic sealing etc. It is important that the situs be securely maintained within the receptacle because a plurality of cartridges 208, each having two piercing cannulas, may be repeatedly inserted and withdrawn from the situs during the useful life of the receptacle 42 and administration set 20. The fluid receiving segment 78 includes a tapered portion 90 below and generally coaxial with the pierceable situs 80. The tapered portion 90 serves as a needle guide into the resilient bushing 92.

The resilient bushing is preferably made of an elastomer such as polyisoprene. The resilient bushing 92 defines a narrow through-bore 94. The resilient bushing is juxtaposed relative to the pierceable situs 80 so that the through-bore 94 is substantially coaxial with the tapered portion 90.

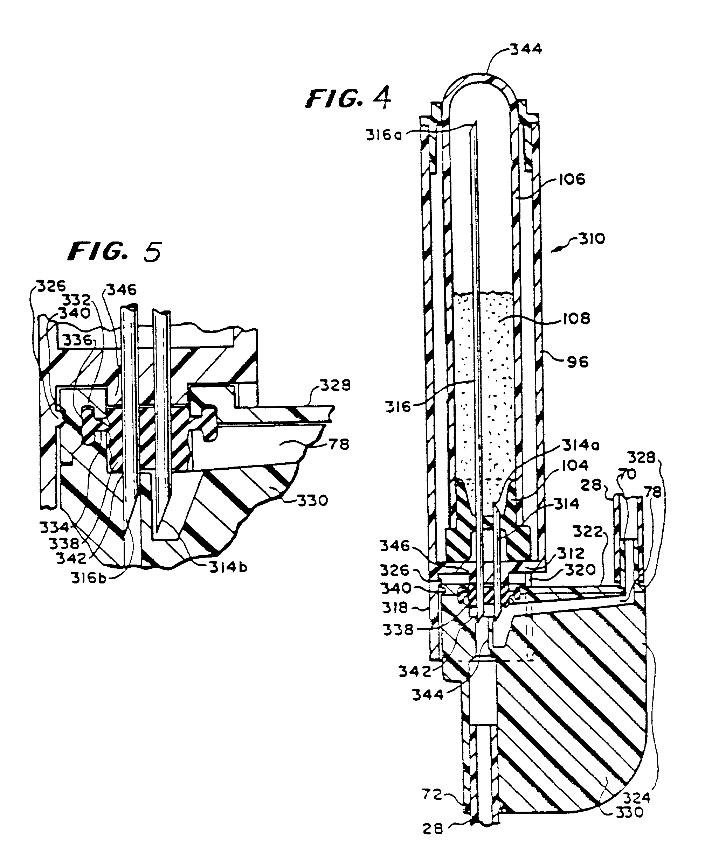

Referring now to Figures 4 and 5, there is illustrated a cartridge 310 (which is not in accordance with the invention), including a rigid cylinder 96 in which is slidably disposed a tubular chamber 106 having beneficial agent 108 therein. A base plate 312 extends across the cylinder 96. Disposed within the base plate 312 are first and second hollow cannulas 314, 316 respectively, each including a first pointed hollow end 314a, 316a respectively facing the tubular chamber 106. The tubular chamber 106 of the cartridge 310 slides from a first position out of engagement with the first and second cannulas to a second position illustrated in Figure 4 where the first ends 314a, 316a of the first and second cannulas 314, 316 have pierced the rubber stopper 104 of the tubular chamber 106.

The cartridge 310 includes a keyway wall 318 extending from the side of the base plate 312 opposite the tubular chamber 106. A keyway slot 320 is defined within the keyway wall 318 for fitting about the bridge 322 of a receptacle 324. The keyway wall 318 includes one or more internal projections 326.

The second pointed hollow ends 314b, 316b of the first and second hollow cannulas 314, 316 respectively may extend the same distance from the base plate 312.

The receptacle 324 includes a receptacle inlet 70 and a receptacle outlet 72 connected to a fluid conduit 28 of an administration set such as the administration set 20. The receptacle 324 includes a fluid receiving segment 78 having an upstream end in flow communication with the inlet 70 and a downstream end in fluid communication with the outlet 72.

The receptacle 324 does not include a bushing such as the bushing 92 in the receptacle 42; however, as will be described below in greater detail, once the cartridge 310 and the receptacle 324 are fully engaged, all liquid flowing through the receptacle must first pass through the tubular chamber 106.

The receptacle 324 includes upper and lower fitments 328, 330 defining an annular channel 332 substantially corresponding to and receiving the ring-like extension 334, including the enlarged periphery 336 thereof, of a pierceable, piston-like injection site 338 made of a resilient pierceable material such as polyisoprene. One or mode detents 340 are provided about the exterior of the receptacle 324 for engagement with the internal projections 326 on the keyway wall 318.

An outflow seal 342 may be molded within the lower fitment 330 of the same relatively rigid plastic material as the remainder of the lower fitment 330. The outflow seal 342 defines an outflow channel 344 of larger diameter than the second hollow cannula 316 of the cartridge 310.

In operation, the nurse or other operator pushes down on the top 344 of the tubular chamber 106, sliding it from the first position to the second position illustrated in Fig. 4 where the stopper 104 abuts the base plate 312. The operator then mounts the cartridge 310 about the receptacle 324 in the only manner permitted by the keyway wall 318, keyway slot 320 and bridge 322. As illustrated in Fig. 4, the cannulas 314, 316 both pierce the situs 338. However, as illustrated in Fig. 4, the cartridge 310 is not fully mounted about the receptacle 324. In Fig. 4, the situs 338 is still in its normal position. Liquid flowing into the inlet 70 may flow through the outlet 72 by passing around the outflow seal 342, without entering the chamber 106.

To completely engage the cartridge and the receptacle, the nurse or other operator pushes down further on the rigid cylinder 96 to reach the fully engaged position illustrated in Fig. 5. By exerting downward pressure on the cartridge 310 relative to the receptacle 324, a central raised portion 346 pushes down on the situs 338 causing it to shift downwardly from its normal position illustrated in Fig. 4 to its second, stressed position illustrated in Fig. 22. The situs moves in a direction substantially mutually orthogonal to the ring-like extension of the situs. In the stressed position shown in Fig. 5, the situs 338 seals about the outflow seal 342 of the receptacle. The stressed position of the injection site 338 is maintained by the interfitment of the now-engaged projections 326 and detents 340.

Liquid now flowing into the inlet 70 and fluid receiving segment 78 is necessarily diverted into the first hollow cannula 314 through end 314b and into the chamber 106 containing the beneficial agent 108. The pressure upon the situs 338 causes an effective liquid seal between the situs 338 and the outflow seal 342. Liquid exits the tubular chamber 106 through the second cannula 316 and subsequently flows out of the receptacle through the outlet 72, downstream to the patient.

The cartridge 310 and receptacle 324 combination eliminate the need for the manufacture and assembly of the bushing to create a single flow path once the cartridge is engaged about the receptacle. After the beneficial agent has been delivered to the patient, the operator may remove the cartridge, whereupon the situs 338 returns to its normal position illustrated in Fig. 4, so that liquid may flow directly through the receptacle. Subsequent cartridges 310 may be secured through the receptacle 324, at which time the situs is once more forced into the stressed position shown in Fig. 5.

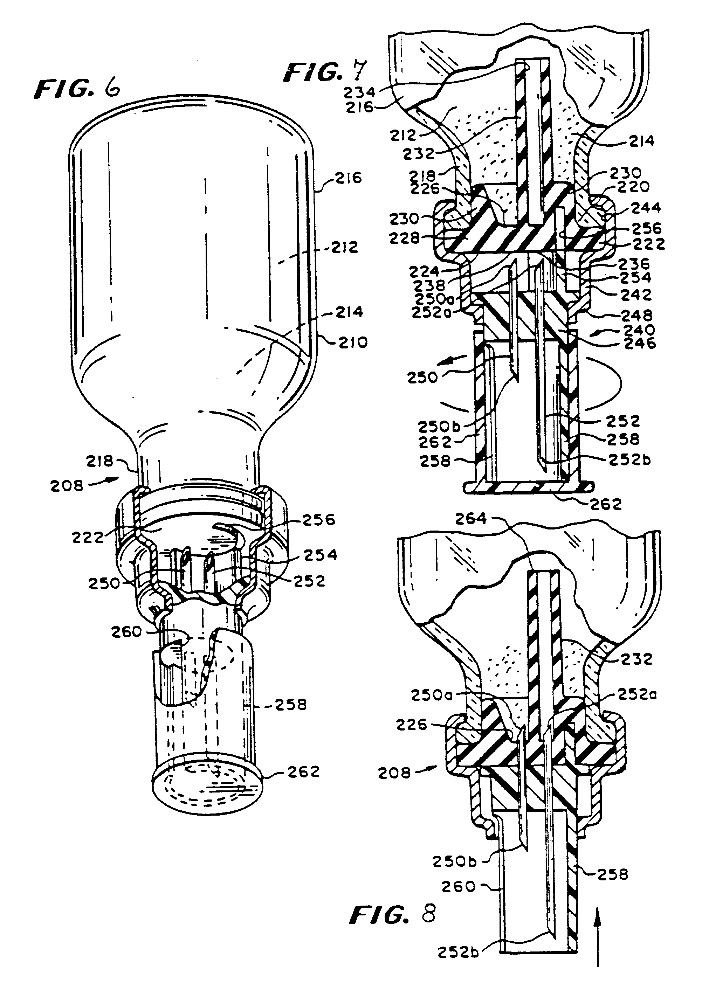

Turning now to Figs. 6 to 8, there is disclosed a cartridge 208 for introducing a beneficial agent into a fluid conduit. The cartridge 208 includes a wall 210 defining a chamber 212 having a beneficial agent 214 therein. The cartridge wall 210 may be a glass drug vial including a body portion 216 and a neck portion 218 having an open end defining a mouth 220. A pierceable closure means such as pierceable stopper 222 is mounted within the mouth 220 and neck 218 of the cartridge 208. The stopper 222 includes an outer face 224 facing the chamber exterior and an inner face 226 facing the defined chamber 212.

The pierceable stopper 222 may include an outer lid portion 228 and a narrower plug portion 230. The lid portion 228 abuts the end of the mouth 220 and the plug portion 230 extends into the neck portion 218 of the chamber 212.

A chimney-like projection 232 extends from the inner face 226 in a direction substantially parallel to the length of the cartridge or, stated differently, in a direction substantially perpendicular to the lid portion 228 of the pierceable stopper 222. The chimney 232, the plug portion 230 and the lid portion 228 may be formed of a single piece of material, such as polyisoprene.

The closure means, in this case the pierceable stopper 222, is adapted to be pierced both at a point in alignment with the interior 234 of the chimney 232 and at a point in alignment with the area of the inner face 226 and external to the chimney 232. These two points are marked by reference numerals 236 and 238 respectively.

In the preferred embodiment, the cartridge further includes a flow connector 240 adapted for mounting about the mouth 220 and closure means of the cartridge. The flow connector includes cartridge connection means such as a sleeve 242 having an enlarged channel 244 at one end thereof for tight interfitment with the mouth 220 and the pierceable stopper 222.

The flow connector 240 further includes a base 246 mounted to the other end 248 of the sleeve 242. It is preferred that the base 246 is rotatably mounted within the sleeve 242.

The flow connector 240 includes first and second cannulas 250, 252 mounted within the base 246. The first and second cannulas include first pointed ends 250a, 252a respectively facing the pierceable stopper. Similarly, the cannulas each include a second pointed end 250b, 252b extending away from the pierceable stopper on the opposite side of the base 246. The cannulas extend in a direction substantially parallel to the length of the chimney 232 and substantially perpendicular to the lid portion 228 of the pieceable stopper 222.

The flow connector 240 further includes a projecting key 254 extending from the stopper facing side of the base 246. A mating keyway 256 is disposed within the outer face 224 of the stopper 222. The location of the key 254 and keyway 256 may of course be reversed. The key and keyway may have an arc design defined by a radius having a center in alignment with the center of the base 246.

The first ends 250a, 252a of the cannulas extend substantially the same distance from the chamber facing side of the base 246. In the preferred embodiment, the second ends 250b, 252b of the cannulas are disposed such that the second cannula second end 252b extends from the chamber distant side of the base further than the first cannula 250.

The base 246 includes an extension wall 258 extending from the chamber distant side thereof, surrounding and spaced from the first and second cannulas. The extension wall 258 includes a defined slot 260 therein. The extension wall 258 and the second ends 250b, 252b are covered with a cap 262 provided to prevent harm to the nurse or other operator and to prevent touch contamination of the cannulas.

The slot 260 within the extension wall 258 serves as a keyway means for enabling proper engagement of the cartridge 208 with a receptacle such as receptacle 42 mounted in the fluid conduit 28 of an administration set 20.

In operation, the nurse or other operator removes the cap 262 from the extension wall 258 and rotates the extension wall until the key 254 and keyway 256 mate, at which time the extension wall 258 and base 246 are urged toward the pierceable stopper, until the hollow cannulas are moved from a first position illustrated in Fig. 7 in which they are spaced from the chamber 212, to a second position illustrated in Fig. 8 in which both the first and second cannulas 250, 252 have pierced the closure means and are in flow communication with the chamber 212. In the second position, the first cannula pierces the inner face 226 of the stopper at a point external to the chimney. The second cannula pierces the stopper so as to have its first end 252a disposed within the chimney 232.

The cartridge 208 is then inserted about the receptacle 42 illustrated in Fig. 1 by mounting the slot 260 over the bridge 130 of the receptacle 42. In this position, the first and second cannulas 250, 252 will be disposed within the receptacle. Liquid flowing into the receptacle will flow into the chamber 212 through the first cannula 250 and mix with the beneficial agent 214 therein. When the liquid rises to the level of the top 264 of the chimney 232, liquid will flow down the chimney 232 through the second cannula 252 and bushing 92 to the patient. Alternatively, the lengths of the cannulas 250, 252 on the stopper distant side of the base 246 can be changed so that the cartridge 208 may be used with the receptacle 324 illustrated in Figs. 4 and 5.

## Claims

1. A parenteral administration set having an inlet end (30) and an outlet end (34) and a fluid flow path between said ends, said path including a portion provided with penetrating means (250,252) including a fluid inlet (250a) in communication with said inlet end (30) and a fluid outlet (252a) in communication with said outlet end (34), and a cartridge (208) mountable in the administration set, the cartridge having a wall (210) defining an elongate chamber (212) containing a beneficial agent (214) and pierceable closure means (222) closing an end of the chamber, the closure means having an outer face (224) and an opposed inner face (226) facing the chamber, the closure means being penetrable by the penetrating means (250,252) to provide for fluid flow upwardly into the chamber and downwardly out of the chamber, characterised in that the closure means (222) is provided with a hollow open-ended member (232), which extends from said inner face (226) into the chamber (212) in a direction substantially parallel to the length of the chamber, and in that the closure means is penetrable by said penetrating means so as to place the fluid outlet (252a) in communication with the member (232), such that in use, fluid flows from the chamber along the member to the outlet end (34).

2. The administration set in accordance with Claim 1, wherein the penetrating means comprises elements (250,252) which pierce the closure means (222) both at a point in alignment with the interior of said member (232) and at a point in alignment with the area of said inner face external to said member.

3. The administration set in accordance with Claim 2, further comprising a flow connector adapted for mounting on said cartridge (208), said flow connector including cartridge connection means (240), and a base (246) mounted on said connection means; and wherein the penetrating means comprises two hollow cannulas (250;252), each pointed at each end, mounted within said base and extending in a direction substantially parallel to said member (232), said base and said hollow cannulas being slidable relative to said pierceable closure means (222), such that in a first position said hollow cannulas are spaced from said chamber (212), and in a second position said first and second cannulas have pierced said closure means, said second cannula (252) piercing said closure means in alignment with said member such that said second cannula is disposed within said member, said first cannula (250) piercing said closure means at a point on said inner face (226) external to said member.

4. The administration set as in Claim 3, said base (246) including a chamber distant side, wherein said second cannula (252) extends from said chamber distant side of said base farther than said first cannula (250).

5. The administration set as in Claim 4, further comprising a chamber facing side of said base (246), said first and second cannulas (250,252) extending substantially the same distance from said chamber facing side.

6. The administration set as in Claim 3, 4 or 5, said base (246) including a chamber distant side, further comprising a cap (262) sealingly mounted over portions of said cannulas (250) extending from the chamber distant side of the base.

7. The administration set as in any one of Claims 3 to 6, further comprising one of a key and a keyway (256) disposed at said outer face (224) of said closure means (222) and the other of said key (254) and keyway (256 provided upon said base (246), wherein said base (246) is rotatable relative to said closure means (222) and wherein said second cannula (252) is in alignment with said member (232) when said key (254) and said keyway (256) are engaged.

## Revendications

1. Ensemble d'administration parentérale comprenant une extrémité d'entrée (30) et une extrémité de sortie (34) ainsi qu'une voie d'écoulement de fluide entre lesdites extrémités, ladite voie comportant une partie munie d'un moyen de pénétration (250, 252) qui inclut une entrée de fluide (250a) en communication avec ladite extrémité d'entrée (30) et une sortie de fluide (252a) en communication avec ladite extrémité de sortie (34), et une cartouche (208) qui peut être montée dans l'ensemble d'administration, la cartouche comprenant une paroi (210) qui définit une chambre allongée (212) qui contient un produit médical (214) et un moyen de fermeture qui peut être percé (222) qui ferme une extrémité de la chambre, le moyen de fermeture comprenant une face externe (224) et une face interne opposée (226) qui fait face à la chambre, le moyen de fermeture pouvant être pénétré par le moyen de pénétration (254, 252) pour assurer un écoulement de fluide vers le haut à l'intérieur de la chambre et vers le bas hors de la chambre, caractérisé en ce que le moyen de fermeture (222) est muni d'un élément creux à extrémité ouverte (232) qui s'étend depuis ladite face interne (226) à l'intérieur de la chambre (212) suivant une direction sensiblement parallèle à la longueur de la chambre, et en ce que le moyen de fermeture peut être pénétré par ledit moyen de pénétration de manière à placer la sortie de fluide (252a) en communication avec l'élément (232) de telle sorte qu'en utilisation, le fluide s'écoule depuis la chambre le long de l'élément jusqu'à l'extrémité de sortie (34).

2. Ensemble d'administration selon la revendication 1, dans lequel le moyen de pénétration comprend des éléments (250, 252) qui percent le moyen de fermeture (222) à la fois en un point aligné avec l'intérieur dudit élément (232) et en un point aligné avec la zone de ladite face interne à l'extérieur dudit élément.

3. Ensemble d'administration selon la revendication 2, comprenant en outre un connecteur d'écoulement prévu pour être monté sur ladite cartouche (208), ledit connecteur d'écoulement incluant un moyen de connexion de cartouche (240), et une base (246) montée sur ledit moyen de connexion ; et dans lequel le moyen de pénétration comprend deux canules creuses (250 ; 252), chacune étant effilée au niveau de chaque extrémité, ces deux canules étant montées dans ladite base et s'étendant suivant une direction sensiblement parallèle audit élément (232), ladite base et lesdites canules creuses pouvant glisser par rapport audit moyen de fermeture qui peut être percé (222) de telle sorte que, dans une première position, lesdites canules creuses soient écartées de ladite chambre (212) et que, dans une seconde position, lesdites première et seconde canules aient percé ledit moyen de fermeture, ladite seconde canule (252) perçant ledit moyen de fermeture en alignement avec ledit élément de telle sorte que ladite seconde canule soit disposée à l'intérieur dudit élément, ladite première canule (250) perçant ledit moyen de fermeture en un point situé sur ladite face interne (226) à l'extérieur dudit élément.

4. Ensemble d'administration selon la revendication 3, ladite base (246) incluant un côté éloigné de la chambre, dans lequel ladite seconde canule (252) s'étend depuis ledit côté éloigné de la chambre de ladite base au-delà de ladite première canule (250).

5. Ensemble d'administration selon la revendication 4, comprenant en outre un côté qui fait face à la chambre de ladite base (246), lesdites première et seconde canules (250, 252) s'étendant sensiblement sur la même distance à partir dudit côté qui fait face à la chambre.

6. Ensemble d'administration selon la revendication 3, 4 ou 5, ladite base (246) incluant un côté éloigné de la chambre, comprenant en outre un couvercle (262) monté de manière étanche sur des parties desdites canules (250) qui s'étendent depuis le côté éloigné de la chambre de la base.

7. Ensemble d'administration selon l'une quelconque des revendications 3 à 6, comprenant en outre une clavette et un logement de clavette (256) disposés du côté de ladite face externe (224) dudit moyen de fermeture (222) et une autre clavette (254) et un logement de clavette (256) prévus sur ladite base (246), dans lequel ladite base (246) peut tourner par rapport audit moyen de fermeture (222) et dans lequel ladite seconde canule (252) est alignée avec ledit élément (232) lorsque ladite clavette (254) et ledit logement de clavette (256) coopèrent.

**Patentansprüche**

1. Verabreichungssatz für parenterale Applikation mit einem Einlaßende (30) und einem Auslaßende (34) und einem Fluidströmungspfad zwischen den Enden, der einen Bereich mit Eindringeinrichtungen (250,251) aufweist, die mit einem Fluideinlaß (250a) in Verbindung mit dem Einlaßende (30) und einem Fluidauslaß (252a) in Verbindung mit dem Auslaßende (34) versehen ist, und mit einer Patrone (208), die in dem Verabreichungssatz anbringbar ist und eine Wand (210) hat, die eine ein heilsames Mittel enthaltende, längliche Kammer (212) bildet und eine ein Ende der Kammer verschließende, durchstechbare Verschlußeinrichtung (222) aufweist, wobei die Verschlußeinrichtung eine Außenfläche (224) und eine dieser gegenüberliegende, der Kammer zugewandte Innenfläche (226) hat, und welche von den Eindringeinrichtungen (250,252) durchdringbar ist, um für eine Fluidströmung nach oben in die Kammer und nach unten aus der Kammer zu sorgen,
dadurch **gekennzeichnet**,

- daß die Verschlußeinrichtung (222) mit einem hohlen Teil (232) mit offenen Enden versehen ist, das sich von der Innenfläche (226) in einer Richtung im wesentlichen parallel zur Länge der Kammer in die Kammer (212) erstreckt,
- und daß die Verschlußeinrichtung von den Eindringeinrichtungen durchdringbar ist, um den Fluidauslaß (252a) mit dem Teil (232) in Verbindung zu bringen, so daß bei der Benutzung Fluid aus der Kammer längs des Teiles zum Auslaßende (34) fließt.

2. Verabreichungssatz nach Anspruch 1,
wobei die Eindringeinrichtungen Elemente (250,252) aufweisen, die die Verschlußeinrichtung (222) sowohl an einer mit dem Inneren des Teiles (232) fluchtenden Stelle als auch an einer mit dem Bereich der Innenfläche außerhalb des Teiles fluchtenden Stelle durchstoßen.

3. Verabreichungssatz nach Anspruch 2,
ferner mit einem zum Anbringen an der Patrone (208) geeigneten Strömungsverbinder, der Patronenanschlußmittel (240) und eine an den Patronenanschlußmitteln angebrachten Basis (246) aufweist;
und wobei die Eindringeinrichtungen zwei Hohlkanülen (250;252) aufweisen, die jeweils an jedem Ende angespitzt, innerhalb des Basisendes angebracht sind und sich in einer Richtung im wesentlichen parallel zu dem Teil (232) erstrecken, wobei die Basis und die Hohlkanülen relativ zu der durchstechbaren Verschlußeinrichtung (222) so verschiebbar sind, daß die Hohlkanülen in einer ersten Stellung einen Abstand von der Kammer (212) haben, und in einer zweiten Stellung die erste und die zweite Hohlkanüle die Verschlußeinrichtung durchstochen haben, wobei die zweite Kanüle (252) die Verschlußeinrichtung derartig in Ausfluchtung mit dem Teil durchsticht, daß die zweite Kanüle sich innerhalb des Teiles befindet, wobei die erste Kanüle (250) die Verschlußeinrichtung an einer Stelle auf der Innenfläche (226) außerhalb des Teiles durchsticht.

4. Verabreichungssatz nach Anspruch 3,
wobei die Basis (246) eine von der Kammer entfernte Seite hat, wobei die zweite Kanüle (252) sich weiter von der von der Kammer entfernten Seite der Basis erstreckt als die erste Kanüle (250).

5. Verabreichungssatz nach Anspruch 4,
ferner mit einer der Kammer zugewandten Seite der Basis (246), wobei sich die erste und zweite Kanüle (250, 252) im wesentlichen über die gleiche Strecke von der der Kammer zugewandten Seite erstrecken.

6. Verabreichungssatz nach Anspruch 3, 4 oder 5,
wobei die Basis (246) eine von der Kammer entfernte Seite hat, ferner mit einer Kappe (262), die abdichtend über Bereichen der Kanülen angebracht ist, welche sich von der von der Kammer entfernten Seite der Basis erstrecken.

7. Verabreichungssatz nach einem der Ansprüche 3 bis 6,
die ferner eine Komponente von einem Keil und einer Keilnut (256) aufweist, die an der Außenfläche (224) der Verschlußeinrichtung (222) angeordnet ist, während die andere Komponente von Keil (254) und Keilnut (256) an der Basis (246) angebracht ist, wobei die Basis (246) relativ zu der Verschlußeinrichtung (222) drehbar ist und wobei die zweite Kanüle (252) in Ausfluchtung mit dem Teil (232) ist, wenn der Keil (254) und die Keilnut (256) in Eingriff miteinander stehen.

FIG.1

FIG.2

FIG.3

EP 0 267 955 B1

FIG. 4

FIG. 5

12

EP 0 267 955 B1

**FIG. 6**

**FIG. 7**

**FIG. 8**

13

FIG. 9